# EUROPEAN PATENT APPLICATION

(11) **EP 4 345 098 A1**
(43) Date of publication of application: **03.04.2024**
(21) Application number: 22199114.4
(22) Date of filing: 30.09.2022
(51) Int. Cl.: C07D 311/80

(54) **CONVERSION OF CANNABIDIOL TO CANNABINOL USING XYLENE**

(71) Applicant: Biotecus, 01108 Vilnius (LT)
(72) Inventor: Voitechovicius, Andzejus, LT-14192 Avizieniai (LT)
(74) Representative: Zaboliene, Reda

(57) **Abstract**

The present invention provides a method of conversion of at least one cannabinol (CBN) from an initial cannabidiol (CBD) mixture of xylene and extraction method. A purity of final product CBN obtained from one CBD is from 80.00% to 99.99%. The yield of CBN is from 40% to 90% of a total weight of mixture.

## Description

### TECHNICAL FIELD

The present invention relates to the field of cannabinoid synthesis, and in particular the chemical processes for making cannabinol (CBN) from cannabidiol (CBD) in the presence of isomeric mixture of xylene.

### BACKGROUND

Cannabinoids are an important class of diverse compounds that interact with brain receptors which are part of the endocannabinoid system. This series of receptors modulate homeostasis throughout the various pathways in the human body.

CBN is very low psychoactive but has other biological properties. There is interest in the application of CBN to products that are intended to help consumers in falling asleep. CBN may also substitute Δ9-tetrahydrocannabinol (Δ9-THC) for anticancer therapy; numerous reports demonstrated anticancer properties of both compounds. CBN can be obtained by long-term exposure of Δ 9-THC to air or Δ9-THC degradation.

After reviewing the literature, it was noticed that in most cases the transformation from CBD to CBN is carried out using toluene as a solvent. Since toluene is an excise good, it was decided to look for another alternative solvent in which the reaction could be carried out.

US202117144846 discloses processes comprising the continuous isolation and purification of cannabinoids and further isomerization of the purified cannabidiol to Δ8-tetrahydrocannabinol (Δ8-THC) and Δ9-THC.

CA2020050804 discloses methods for converting cannabidiol, cannabidiolic acid and analogues thereof into Δ8-tetrahydrocannabinol, Δ8-tetrahydrocannabinolic acid and analogues thereof. US11292779B relates to the field of cannabinoid synthesis. More specifically, the process described covers industrial scale isomerization of CBD into an essentially pure Δ8-THC extract. US202063007999 discloses a method for converting a cannabidiol-like (CBD-like) starting material to one or more cannabinoids, in which a CBD-like starting material is provided and CBD-like starting material is exposed to an acid reagent in the absence of solvent, or CBD-like starting material is exposed to an oxidant reagent in the absence of solvent, or the CBD-like starting material is exposed to an oxidant reagent and an acid reagent in the absence of solvent, or the CBD-like starting material is exposed to an oxidant reagent that also behaves as an acid in the absence of solvent, to yield one or more cannabinoid products.

The goal of this invention is to use alternative, not expensive basic solvent for the conversion reaction. Experimental data shows that isomeric mixture of xylene, including *para*-xylene, *meta*-xylene, *ortho-*xylene is the most appropriate solvent.

The main objectives of a present invention are to optimise reaction temperature, the term of reaction, and choose more appropriate reagent. The optimisation was aimed, because no toxic compounds such as chromates, borates, etc. were used and there were no heavy metals in the final product. In addition, the CBN was obtained by using the least number of compounds catalysing reaction with less additional contamination of the target product.

### BRIEF DESCRIPTION OF THE INVENTION

The present invention provides a method of conversion of at least one cannabinol (CBN) from cannabidiol (CBD) using isomeric mixture of xylene and extraction method. A purity of final product CBN obtained from one CBD is from 80.00% to 99.99%. The yield of CBN is from 40% to 90% of a total weight of mixture.

The method of conversion of cannabidiol (CBD) into cannabinoids (CBN) comprises following steps: a) mixing isomeric mixture of xylene with cannabidiol isolate to form a xylene-cannabidiol mixture; b) dissolving CBD in mixture by heating; c) adding iodine and water to the mixture; d) refluxing of mixture for 12-16 days with heating until the amount of intermediate compounds are less than 1,5%; e) separating and washing of the organic part of solution; f) crystallization of the solution; g) filtering the crystals from the mixture of solvents; and h) drying of the crystals.

Isomeric mixture of xylene used in step a) is selected from *ortho-, meta-* and *para-* xylene. CBD in mixture is dissolved at temperature of 80°C and further refluxing is performed for 24 hours at temperature between 105 and 110°C, iodine is used as reagent. The separated organic layer is washed at least three times and crystallization is performed at least two times. The crystallisation is performed using solvent mixture of toluene: hexane in a ratio of 1:4. Drying is performed in vacuum at temperature between 40 and 55°C.

### BRIEF DESCRIPTION OF DRAWINGS

Fig. 1 illustrates a chromatogram of starting compound CBD.
Fig. 2 illustrates the chromatogram of the conversion from CBD to CBN(Ml).
Fig. 3 illustrates the chromatogram of the conversion from CBD to CBN (M2).
Fig. 4 illustrates the chromatogram of CBN when reaction is stopped (M3).
Fig. 5 illustrates the chromatogram of the final CBN product after distillation and purification.

### DETAILED DESCRIPTION OF THE INVENTION

Stated herein is the preferred method for converting CBD to CBN which is illustrated in formula I:

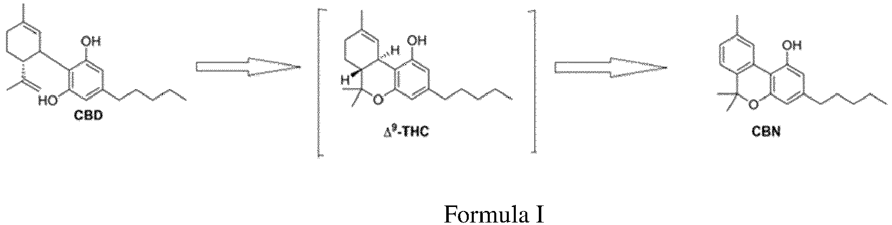

The reaction mixture can be manipulated by time, temperature, and catalyst concentration to produce dried crystals of CBN in different purities.

Further objects and advantages will become apparent upon reading the following detailed description of an embodiment of the invention.

200g of CBD is added to 1 L three-necked flask with a magnetic stirrer, 400 ml of xylene (a mixture of isomers) is poured, and heating is turned. The flask is placed in a glycerine bath, setting the temperature to 80°C at the beginning. After the CBD has dissolved, 3g of iodine and 1.2 ml of water are added to the flask and the reaction is performed at reflux.

The reaction temperature is set between 105 and 110°C (at a temperature of the reaction mixture higher than 110°C, unwanted impurities start to form in the reaction mixture and below 105°C the reaction is difficult) and the reaction mixture is heated until it becomes a slightly yellowish solution. The progress of the reaction is monitored using high-performance liquid chromatography (HPLC). The reaction is terminated when the amounts of intermediate compounds Δ8-THC, Δ9-THC, and CBC in the reaction mixture are less than 1.5% of each individually. The reaction takes place for 12-16 days.

The conversion of the reaction is 80-85% of CBN, after which the reaction mixture is cooled to 80°C and air bubbles stop. 350 ml of water is poured into the reaction mixture and 15 g of sodium sulphite (Na₂SO₃) is added. Then reaction mixture is stirred for 24 hours at a temperature of 80°C.

After a day the reaction mixture is poured into a separating funnel and the organic layer is separated. The organic layer is washed 3 times with 350 ml of water. The washed organic layer is concentrated from xylene using a rotary evaporator. The concentrated reaction mixture is distilled in vacuum at 210°C/1.5 mbar, the resulting distillate is dissolved in hexane in a ratio of 1:3 and placed at 0°C temperature. The resulting crystals are filtered and recrystallized from a solvent mixture of toluene:hexane in a ratio of 1:4. The obtained solution is placed at a temperature of 0°C. The obtained crystals are filtered and if the purity of the crystals is less than 98%, they are recrystallized from a toluene:hexane mixture. The resulting white crystals are dried in a vacuum at 40°C and then at 55°C until the reaction yield reaches 65-70%.

After three crystallizations white crystals are obtained with a melting point of 77-78°C, with no extraneous odour, purity according to HPLC reaches 98.4% of the target product CBN.

### Experimental studies

Several experimental studies were performed which provides positive results in conversion from CBD to CBN and obtaining the final CBN product.

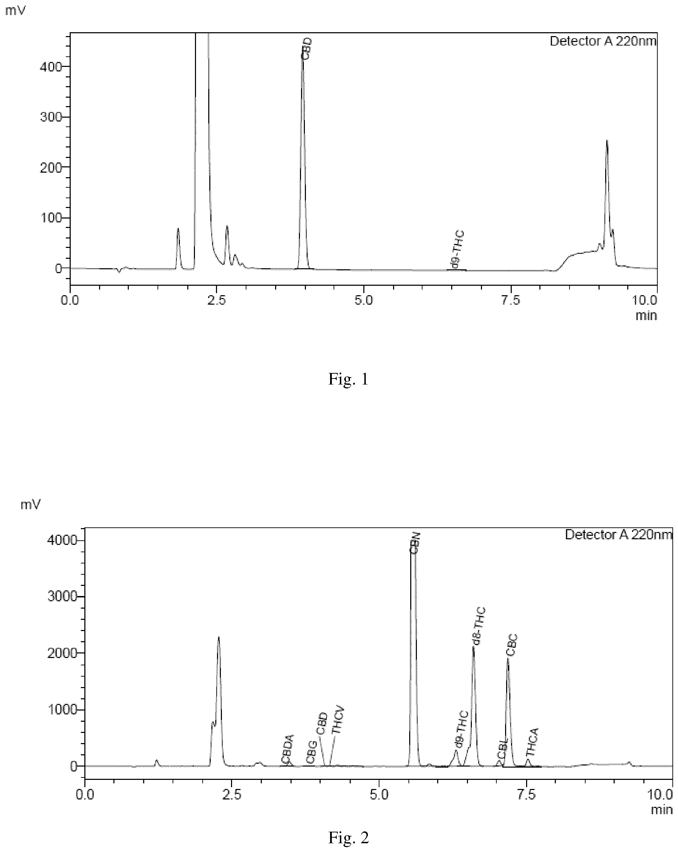

A starting compound is essentially pure compound. The starting compound CBD was used whose purity is more than 99.5% (Table 1, Fig. 1).

**Table 1**

| Detector | QuantitativeResult 3252022 CBD prad_001.lcd A | | | | | | | |
|---|---|---|---|---|---|---|---|---|
| Name | Ret. Time | Height | Area | Dry weight % | Area% | Conc. | Unit | Conc.% |
| CBDV | -- | -- | -- | 0.00 | -- | -- | mg/L | -- |
| CBDA | -- | -- | -- | 0.00 | -- | | -- mg/L | -- |
| CBGA | -- | -- | -- | 0.00 | -- | | -- mg/L | -- |
| CBG | -- | -- | -- | 0.00 | -- | | -- mg/L | -- |
| CBD | 3.961 | 441048 | 1977028 | 19248.97 | 99.771 | 192.490 | mg/L | 100.000 |
| THCV | -- | -- | -- | 0.00 | -- | | -- mg/L | -- |
| CBN | -- | -- | -- | 0.00 | -- | | -- mg/L | -- |
| d9-THC | 6.500 | 344 | 4537 | 0.00 | 0.229 | 0.000 | mg/L | 0.000 |
| d8-THC | -- | -- | -- | 0.00 | -- | | -- mg/L | -- |
| CBL | -- | -- | -- | 0.00 | -- | | -- mg/L | -- |
| | | | | | | | | |

| Name | Ret. Time | Height | Area | weight | Area% | Conc. | Unit | Conc.% |
|---|---|---|---|---|---|---|---|---|
| CBC | -- | -- -- | | Dry % 0.00 | -- | -- | mg/L | -- |
| THCA | -- | -- -- | | 0.00 | -- | -- | mg/L | -- |
| Total THC | 0.00 | | | | | | | |
| Total THC | 0.00 | | | | | | | |
| Total CBD | 19248.97 | | | | | | | |
| Total CBD | 192489.70 | | | | | | | |

The reaction steps are illustrated in M1, M2 and M3 reports.

M1 report shows the first step in which the conversion from CBD to CBN reaches more than 53% (Table 2, fig. 2).

**Table 2**

| Detector A | QuantitativeResult 3252022_M1_003.lcd | | | | | | | |
|---|---|---|---|---|---|---|---|---|
| Name | Ret. Time | Height | Area | Dry weight % | Area% | Conc. | Unit | Conc.% |
| CBDV | -- | -- | -- | 0.00 | -- | -- | mg/L | -- |
| CBDA | 3.371 | 8623 | 24497 | 209.95 | 0.052 | 2.099 | mg/L | 0.090 |
| CBGA | -- | -- | -- | 0.00 | -- | -- | -mg/L | -- |
| CBG | 3.794 | 6850 | 30391 | 312.22 | 0.064 | 3.122 | mg/L | 0.134 |
| CBD | 4.079 | 3062 | 10337 | 104.85 | 0.022 | 1.049 | mg/L | 0.045 |
| THCV | 4.162 | 4366 | 13442 | 100.39 | 0.028 | 1.004 | mg/L | 0.043 |
| CBN | 5.548 | 3998182 | 25528187 | 141566.58 | 53.809 | 1415.666 | mg/L | 60.709 |
| d9-THC | 6.312 | 285889 | 1514783 | 0.00 | 3.193 | 0.000 | mg/L | 0.000 |
| d8-THC | 6.608 | 2108492 | 10584621 | 0.00 | 22.310 | 0.000 | mg/L | 0.000 |
| CBL | 7.046 | 97576 | 351581 | 2812.12 | 0.741 | 28.121 | mg/L | 1.206 |
| | | | | | | | | |

| Name | Ret. Time | Height | Area | Dry weight % | Area% | Conc. | Unit | Conc.% |
|---|---|---|---|---|---|---|---|---|
| CBC | 7.192 | 1919078 | 8223701 | 83822.05 | 17.334 | 838.220 | mg/L | 35.946 |
| THCA | 7.530 | 126925 | 483976 | 4259.40 | 1.020 | 42.594 | mg/L | 1.827 |
| Total THC | | | | | | | | 73511.93 |
| Total THC | | | | | | | | 735119.34 |
| Total CBD | | | | | | | | 288.97 |
| Total CBD | | | | | | | | 2889.73 |

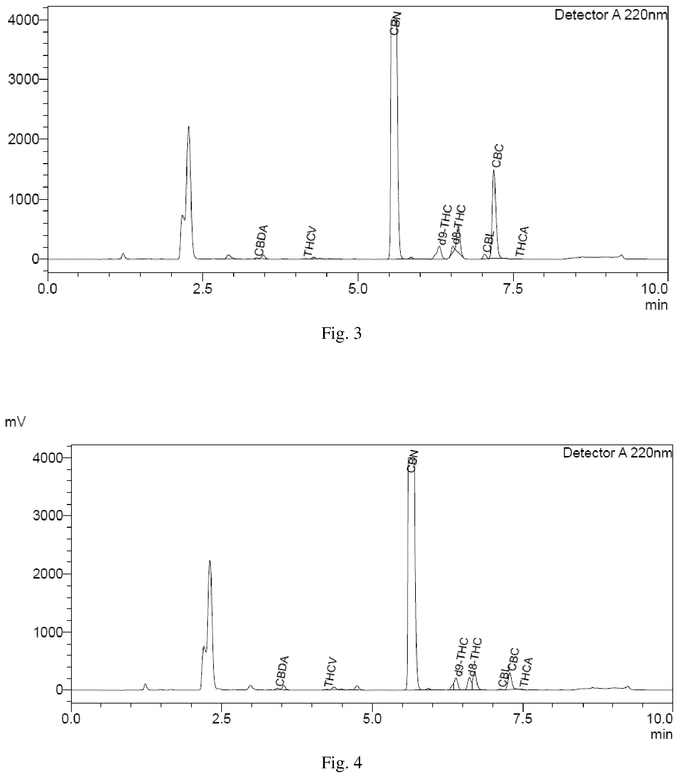

M2 report shows the second step in which the conversion from CBD to CBN reaches more than 73% (Table 3, Fig. 3).

**Table 3**

| Detector A | QuantitativeResult 3282022_M2_003.lcd | | | | | | | |
|---|---|---|---|---|---|---|---|---|
| Name | Ret. Time | Height | Area | Dry weight % | Area% | Cone. | Unit | Conc. % |
| CBDV | -- | -- | -- | 0.00 | -- | -- | mg/L | -- |
| CBDA | 3.367 | 16586 | 55968 | 477.89 | 0.142 | 4.779 | mg/L | 0.211 |
| CBGA | -- | -- | -- | 0.00 | -- | -- | mg/L | -- |
| CBG | -- | -- | -- | 0.00 | -- | -- | mg/L | -- |
| CBD | -- | -- | -- | 0.00 | -- | -- | mg/L | -- |
| THCV | 4.162 | 8744 | 31286 | 255.19 | 0.080 | 2.552 | mg/L | 0.113 |
| CBN | 5.542 | 3999722 | 28816197 | 159800.67 | 73.359 | 1598.007 | mg/L | 70.558 |
| d9-THC | 6.311 | 213044 | 1120657 | 0.00 | 2.853 | 0.000 | mg/L | 0.000 |
| d8-THC | 6.533 | 98394 | 290266 | 0.00 | 0.739 | 0.000 | mg/L | 0.000 |
| CBL | 7.045 | 80743 | 283967 | 2271.76 | 0.723 | 22.718 | mg/L | 1.003 |

| Name | Ret. Time | Height | Area | Dry weight % | Area% | Cone. | Unit | Conc.% |
|---|---|---|---|---|---|---|---|---|
| CBC | 7.191 | 1479527 | 6198497 | 63180.59 | 15.780 | 631.806 | mg/L | 27.897 |
| THCA | 7.568 | 9359 | 55679 | 493.60 | 0.142 | 4.936 | mg/L | 0.218 |
| Total THC | | | | | | | | 55409.38 |
| Total THC | | | | | | | | 554093.81 |
| Total CBD | | | | | | | | 419.11 |
| Total CBD | | | | | | | | 4191.11 |

The reaction is stopped at 84% (M3) conversion as the intermediates substantially are converted to the target product CBN (Table 4, Fig. 4).

**Table 4**

| | QuantitativeResult 3292022_M3_003.lcd Detector A | | | | | | | |
|---|---|---|---|---|---|---|---|---|
| Name | Ret. Time | Height | Area | Dry weight % | Area% | Cone. | Unit | Conc.% |
| CBDV | -- | -- | -- | 0.00 | -- | -- | mg/L | -- |
| CBDA | 3.428 | 24334 | 85445 | 728.85 | 0.232 | 7.289 | mg/L | 0.389 |
| CBGA | -- | -- | -- | 0.00 | -- | -- | mg/L | -- |
| CBG | -- | -- | -- | 0.00 | -- | -- | mg/L | -- |
| CBD | -- | -- | -- | 0.00 | -- | -- | mg/L | -- |
| THCV | 4.244 | 11946 | 42967 | 356.53 | 0.116 | 3.565 | mg/L | 0.191 |
| CBN | 5.608 | 3998677 | 31296115 | 173553.38 | 84.832 | 1735.534 | mg/L | 92.735 |
| d9-THC | 6.393 | 210014 | 810142 | 0.00 | 2.196 | 0.000 | mg/L | 0.000 |
| d8-THC | 6.619 | 215129 | 854397 | 0.00 | 2.316 | 0.000 | mg/L | 0.000 |
| CBL | 7.131 | 15065 | 51278 | 412.15 | 0.139 | 4.122 | mg/L | 0.220 |
| | | | | | | | | |

| Name | Ret. Time | Height | Area | Dry weight % | Area% | Cone. | Unit | Conc.% |
|---|---|---|---|---|---|---|---|---|
| CBC | 7.283 | 289877 | 1167019 | 11898.36 | 3.163 118.984 | | mg/L | 6.358 |
| THCA | 7.483 | 8777 | 22363 | 200.67 | 0.061 | 2.007 | mg/L | 0.107 |
| Total THC | | | | | | | | 10434.86 |
| Total THC | | | | | | | | 104348.65 |
| Total CBD | | | | | | | | 639.20 |
| Total CBD | | | | | | | | 6392.04 |

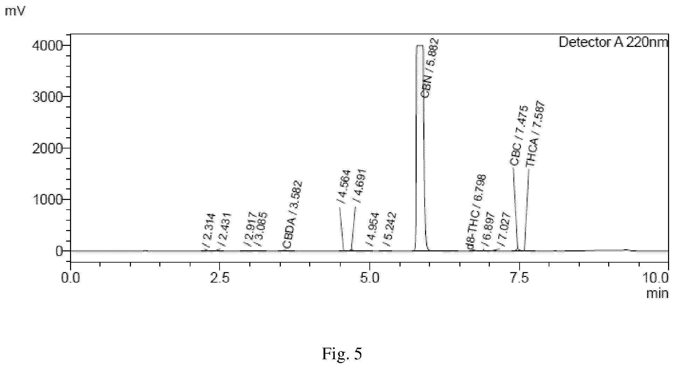

This report provides the final CBN product after distillation and purification, the purity of this product is 98.4%. The crystallisation was performed 3 times in hexane. The white crystals are obtained with a melting temperature of 77-78°C and a reaction yield of 60-65% (Table 5, Fig. 5).

**Table 5**

| QuantitativeResult 6172022_3k Hex+T**_**005.lcd | | | | | | | | |
|---|---|---|---|---|---|---|---|---|
| Detector A | | | | | | | | |
| Name | Ret. Time | Height | Area | Dry weight % | Area% | Conc. | Unit | Conc.% |
| CBDV | -- | -- | -- | 0.00 | -- | -- | mg/L | -- |
| CBDA | 3.582 | 16162 | 69757 | 595.29 | 0.211 | 5.953 | mg/L | 0.325 |
| CBGA | -- | -- | -- | 0.00 | -- | -- | mg/L | -- |
| CBG | -- | -- | -- | 0.00 | -- | -- | mg/L | -- |
| CBD | -- | -- | -- | 0.00 | -- | -- | mg/L | -- |
| THCV | -- | -- | - | 0.00 | -- | -- | mg/L | -- |
| CBN | 5.882 | 3999249 | 32590670 | 180732.50 | 98.435 | 1807.325 | mg/L | 98.647 |
| d9-THC | -- | -- | -- | 0.00 | -- | -- | mg/L | -- |
| d8-THC | 6.798 | 3038 | 14551 | 0.00 | 0.044 | 0.000 | mg/L | 0.000 |
| CBL | -- | -- | -- | 0.00 | -- | -- | mg/L | -- |
| | | | | | | | | |

| Name | Ret. Time | Height | Area | Dry weight % | Area% | Conc. | Unit | Conc.% |
|---|---|---|---|---|---|---|---|---|
| CBC | 7.475 | 39548 | 163824 | 1673.53 | 0.495 | 16.735 | mg/L | 0.913 |
| THCA | 7.5871 | 6004 | 23346 | 209.31 | 0.071 | 2.093 | mg/L | 0.114 |
| Total THC | | | | | | | | 1467.68 |
| Total THC | | | | | | | | 14676.82 |
| Total CBD | | | | | | | | 522.07 |
| Total CBD | | | | | | | | 5220.68 |

Therefore, the present disclosure is well adapted to attain the aim and advantages mentioned as well as those that are inherent therein. The particular embodiments disclosed above are illustrative only, as the present disclosure may be modified and practiced in different but equivalent manners apparent to those skilled in the art having the benefit of the description herein. Although individual embodiments are discussed, the disclosure covers all combinations of all those embodiments.

## Claims

1. A method of conversion of cannabidiol (CBD) into cannabinol (CBN), wherein the method comprises:
a) mixing isomeric mixture of xylene with cannabidiol isolate to form a xylene-cannabidiol mixture;
b) dissolving CBD in mixture by heating;
c) adding iodine and water to the mixture;
d) refluxing of mixture with heating until amount of intermediate compounds are less than 1,5%;
e) separating and washing of the organic part of solution;
f) crystallization of the solution;
g) filtering the crystals from the mixture of solvents; and
h) drying of the crystals.

2. The method according to claim 1, wherein isomeric mixture of xylene used in step a) is selected from *ortho-, meta-* and *para-* xylene.

3. The method according to claim 1 or 2, wherein dissolving of CBD in mixture is at temperature of 50-100°C.

4. The method according to any one of claim 1 to 3, wherein in step e) the solution is washed at least three times.

5. The method according to any one of claim 1 to 4, wherein the refluxing is performed at temperature of 105-110°C.

6. The method according to any one of claims 1 to 5, wherein the reagent is iodine.

7. The method according to any one of claims 1 to 6, wherein the step f. crystallization is performed at least two times.

8. The method according to any one of claims 1 to 7, wherein solvent mixture used in the step of crystallization is toluene and hexane.

9. The method according to claim 8, wherein the ratio of toluene and hexane in solvent mixture is 1:4.

10. The method according to any one of claims 1 to 9, wherein drying is performed in vacuum at temperature between 40 and 55°C.

11. The method according to any one of claims 1 to 10, wherein purity of final product CBN obtained from one CBD is from 80.00% to 99.99%.

12. The method according to any one of claims 1 to 12, wherein yield of CBN is from 40% to 90% of a total mixture weight.
